# EUROPEAN PATENT SPECIFICATION

(11) **EP 0 725 674 B1**
(45) Date of publication and mention of the grant of the patent: **10.09.1997**
(21) Application number: 94915729.1
(22) Date of filing: 06.05.1994
(51) Int. Cl.: B01F 15/02, A61F 2/46

(54) **METHOD AND DEVICE FOR FEEDING COMPONENTS FOR BONE CEMENT INTO A MIXING VESSEL FOR THESE**
VERFAHREN UND VORRICHTUNG ZUM ZUFÜHREN VON BESTANDTEILEN VON KNOCHENZEMENT IN EINEN MISCHBEHÄLTER,WO SIE GEMISCHT WERDEN
PROCEDE ET DISPOSITIF PERMETTANT D'INTRODUIRE DES CONSTITUANTS D'UN CIMENT POUR LES OS DANS UN RECIPIENT OU ILS DOIVENT ETRE MELANGES

(30) Priority: 10.05.1993 SE 9301599
(43) Date of publication of application: 14.08.1996
(73) Proprietor: CEMVAC SYSTEM AKTIEBOLAG, 589 41 Linköping (SE)
(72) Inventor: JONSSON, Sören, S-582 70 Linköping (SE)
(74) Representative: Willquist, Bo
(86) International application number: PCT/SE94/00415
(87) International publication number: WO 94/26403

(56) References cited:
- US-A- 4 973 168

## Description

The present invention relates to a method for successively feeding batches of constituent components into a mixing vessel for the preparation of bone cement under vacuum. The invention also relates to an arrangement for successively feeding batches of constituent components into a mixing vessel under partial vacuum for the preparation of bone cement.

Bone cement is prepared by mixing polymethyl methacrylate in powder form and liquid monomethyl methacrylate in a mixing container. Both the liquid component and the mixture give off substances in gaseous form which are environmentally harmful and injurious to the health. It is important for this reason for both the introduction of the bone cement components into the mixing container and the mixing process itself to take place in such a way that the smallest possible quantity of the gases that are injurious to the health escapes into the surrounding environment. Mixing vessels by means of which both the introduction of the components and the preparation of bone cement can be performed without the release of the aforementioned gases to a significant degree are described in SE-C-8901599-4 and SE-A0-9201353-1, for example.

In order for the bone cement to develop optimal strength during use, it is also important for the components contained in the cement to have predetermined proportions.

The object of the present invention is to make available a method of the kind described by way of introduction, which avoids the risk of gas release when feeding the bone cement components into the mixing vessel. This is achieved in accordance with the invention in that, according to the method and in an arbitrary sequence, a glass ampoule containing a liquid bone cement component surrounded by an inner container communicating with the atmosphere and with the mixing vessel is opened so that the contents of the ampoule, under the effect of the partial vacuum inside the mixing vessel, can be sucked down into it, in that a space formed by the spaces between the aforementioned inner container and by the outer container that encloses it at least partially and filled with a bone cement component in powder form is caused by displacement of the inner container relative to the outer container to move from a first position in which the space does not communicate with the atmosphere or the mixing vessel to a second position in which it communicates with the atmosphere and the mixing vessel, so that the bone cement component in powder form can be sucked down into the mixing vessel under the effect of the partial vacuum inside it.

An arrangement for carrying out the method in accordance with the invention is characterized in that it comprises, on the one hand, an inner container communicating with the atmosphere, which is so arranged as to enclose a glass ampoule containing a liquid bone cement component, and as to communicate with the aforementioned mixing vessel, and comprising means for opening the ampoule so that its contents, under the effect of the partial vacuum inside the mixing vessel, can be sucked down into it and, on the other hand, an outer container enclosing the inner container at least partially and so arranged as to communicate with the mixing vessel, which outer container, together with the inner container, defines a space filled with a certain quantity of a bone cement component in powder form, and in which the inner container is capable of displacement from a first position, in which sections of the inner container prevent communication between both the mixing vessel and the atmosphere, to a second position, in which communication between the mixing vessel on the one hand and the atmosphere on the other hand is open, so that the bone cement component in powder form, under the effect of the partial vacuum inside the mixing vessel, can be sucked down into it.

Especially advantageous forms of the arrangement in accordance with the invention can be appreciated from the relevant Patent Claims.

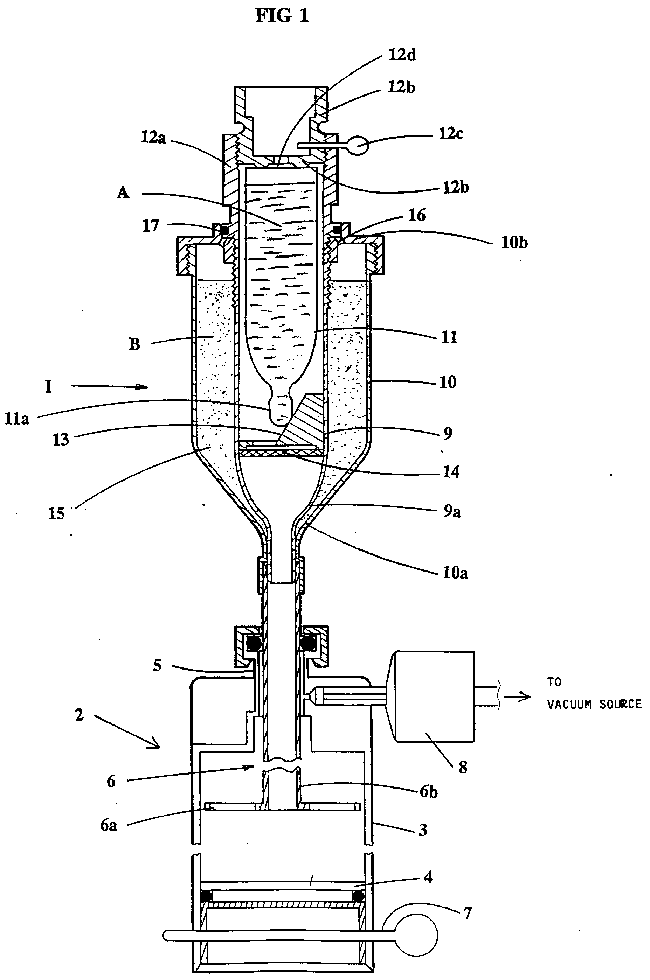

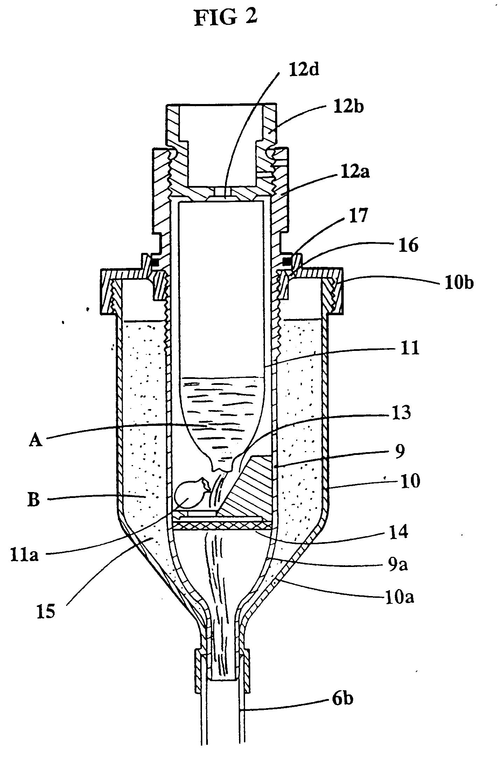

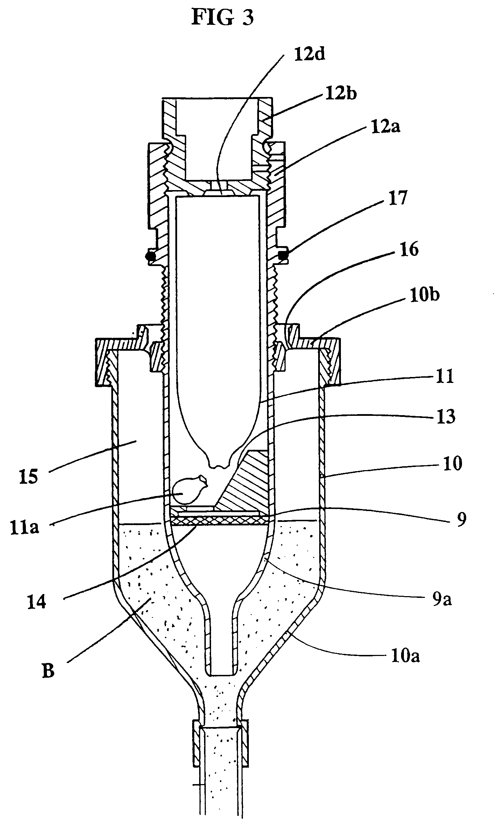

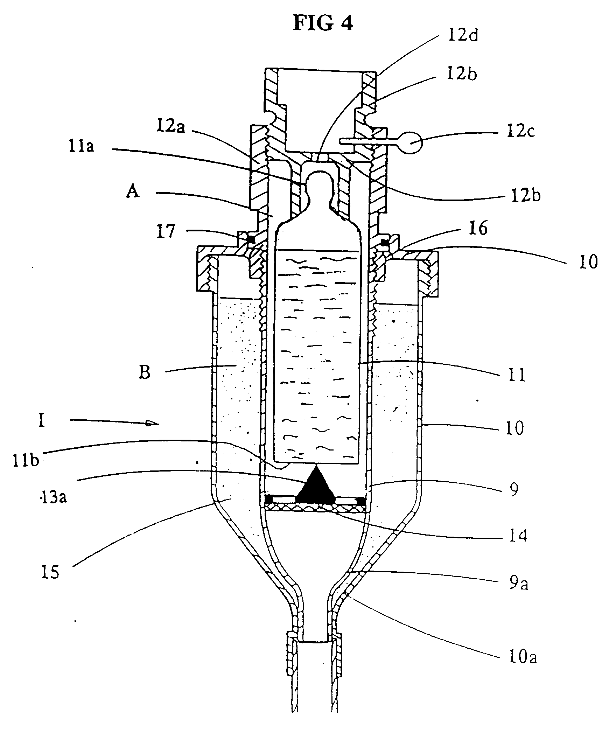

The invention is described below in greater detail with reference to the accompanying drawing, in which Fig. 1 illustrates schematically in longitudinal section an embodiment of a feed arrangement in accordance with the invention filled with bone cement components in liquid and powder form respectively and attached to a mixing vessel. Fig. 2 illustrates the feeding of the liquid bone cement component into the mixing vessel, and Fig. 3 illustrates the feeding of the bone cement component in powder form into the mixing vessel. Figures 4-6 illustrate, in a corresponding fashion to Figures 1-3, an alternative embodiment of a feed arrangement in accordance with the invention.

The designations 1 and 2 are used generally in the drawing in respect of a feed arrangement and a mixing vessel. The former comprises a cylindrical container 3 with a bottom 4 at one end and a neck 5 with an opening at the other end, together with an agitator 6 capable of axial movement in the container 3 consisting of an agitator disc 6a and a tubular agitator rod 6b. This is mounted so that it is free to slide and to produce a seal in the neck 5, in such a way that the agitator 6 can be used to bring about mixing of the bone cement components. Once mixing is complete, and once a lock 7 has been removed, the bottom 4 can be displaced axially in the manner of a piston towards the neck 5 for the purpose of discharging the bone cement via the agitator rod 6b, which now serves as a discharge nozzle. The interior of the container 3 communicates via a filter 8 with a vacuum source (not shown) during feeding of the bone cement components and during mixing of these. Rapid and effective feeding of the bone cement components into the mixing vessel, and safe handling of the gases that are environmentally harmful and injurious to the health, are achieved in this way.

The feeding of the bone cement components from the feed arrangement 1 into the mixing vessel takes place via the agitator rod 6b in a way that will be described later.

The feed arrangement 1 comprises an inner, essentially cylindrical container 9 communicating with the atmosphere and an outer, similarly essentially cylindrical container 10 enclosing the inner container at least partially. The container 9 is so arranged as to enclose a glass ampoule 11 containing the liquid bone cement component, and as to communicate with the mixing vessel 2, as already mentioned, via its agitator rod 6b. For the purpose of breaking the ampoule 11, the container 9 has a cylindrical part 12a at the end situated at the top in the drawing with a bottom 12b capable of axial displacement relative to it.

In the embodiment illustrated in the drawing, the facility for displacement to take place between the cylindrical part 12a and the bottom 12b is achieved by means of threads engaging in one another, in conjunction with which the bottom 12b has an opening 12d between the interior of the container 9 and the atmosphere, and the container 9 has a funnel-shaped part 9a at the end situated at the bottom in the drawing, the narrowest part of which discharges above the agitator rod 6b. In the embodiment in accordance with Figures 1-3, the tip of the glass ampoule 11 points downwards, and there is present inside the inner container 9 an oblique plane 13, against which the tip 11a of the glass ampoule 11, which has a fractural impression, rests. As the bottom 12b of the inner container 9 is screwed downwards, the tip 11a is eventually broken off against the oblique plane 13, and the contents of the ampoule are sucked down into the mixing vessel 2 by the partial vacuum inside it, as illustrated in fig. 2. A filter 14 is provided for the purpose of preventing glass splinters from the glass ampoule 11 from accompanying its contents down into the mixing vessel 2. In the embodiment in accordance with Figures 4-6, the tip 11a of the glass ampoule points upwards, and the inner container 9 has an upward-facing pointed cone 13a, against which the bottom 11b of the glass ampoule rests. As the bottom 12b of the inner container 9 is screwed downwards, the bottom 11b of the glass ampoule 11 is penetrated by the cone 13a and, as previously described for the embodiment in accordance with Figures 1-3, the contents of the glass ampoule are sucked down into the mixing vessel 2. As the liquid bone cement component in the glass ampoule 11 is flowing down into the mixing vessel 2, air is sucked in via the opening 12d, thereby preventing the gases from the liquid bone cement component from finding their way into the atmosphere.

The outer container 10 is essentially identical in shape to the inner container 9, is so arranged as to be capable of being in contact with the mixing vessel 2 in a similar fashion to the inner container 9, has a lower funnel-shaped part 10a, and has a diameter larger than the inner container 9, so that a space 15 for the bone cement component in powder form is formed between the containers 9, 10. The bottom 10b of the outer container 10 is in threaded engagement with the inner container 9, so that the latter can be displaced axially from a position shown in Figures 1 and 2, in which the funnel-shaped parts 9a, 10a of the containers 9, 10 form a closure of the space 15 to a position shown in Fig. 3, in which the space 15 communicates with the inside of the mixing vessel. In the latter position, a duct 16 is opened so that the space 15 also communicates with the atmosphere, for the purpose of facilitating the introduction of the bone cement component in powder form achieved through the partial vacuum inside the mixing vessel, and of preventing the aforementioned gases from finding their way into the atmosphere. An annular seal 17 provides sealing in the first-mentioned position between the upper container 9 and the bottom 10b of the outer container 10.

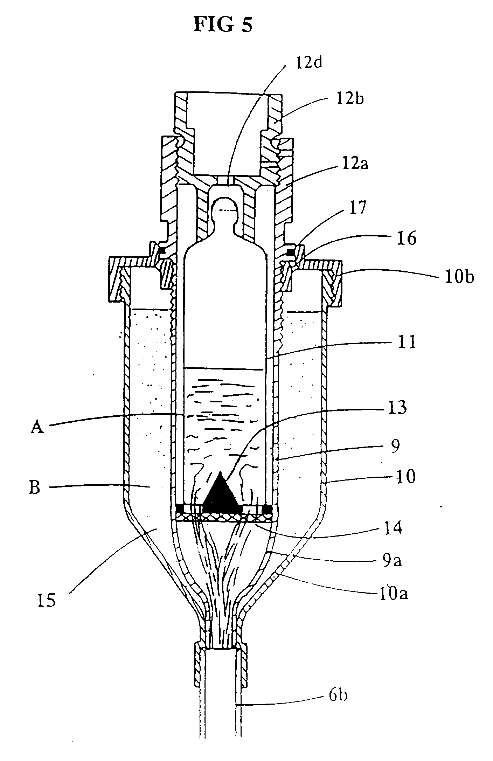

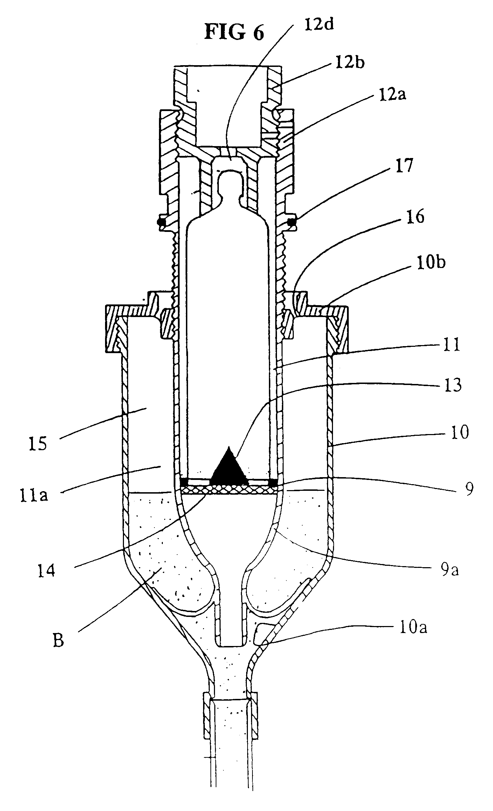

The embodiment in accordance with Figure 6 differs from the embodiment in Figures 4, 5 through brush-shaped devices so arranged as to make contact with the surface of the funnel-shaped part 10a. The bone cement component B is released from the aforementioned surface by relative rotation between the outer and inner container.

A feed procedure is now summarized below with reference to the Figures in the drawing.

The feed arrangement in accordance with the invention is supplied ready for use, i.e. filled with the bone cement components in the correct proportions.

In order to permit feeding of the bone cement components into the mixing vessel 2 from the feed arrangement 1 in accordance with the invention, the mixing vessel 2 is required to be connected to an active vacuum source. The pin 12c is first removed, and the displaceable bottom 12b is screwed downwards, in conjunction with which the glass ampoule is caused to move downwards. Screwing continues until the tip 11a of the glass ampoule 11 is broken off against the oblique plane 13, see Fig. 2, or until the bottom 11b of the glass ampoule is penetrated by the tip of the cone 13a; see Fig. 5. The liquid bone cement component now flows down into the mixing chamber 2 under the effect of the partial vacuum inside it. Once the glass ampoule 11 is totally empty, the cylindrical part 12a is rotated so that the inner container 9 is displaced upwards from the position shown in Figures 1 and 2, in which the space 15 containing the bone cement component in powder form is closed both at the top and at the bottom, to the position shown in Figure 3, in which the aforementioned space is open both at the top, via the duct 16, and at the bottom. Once the space 15 has been completely emptied, the feed arrangement 1 is removed, and the inner tubular part of the agitator rod is sealed with a sealing rod which seals against the mouth of 6a. The mixing procedure can now start.

The feed arrangement in accordance with the invention can be modified in many ways within the scope of the idea of invention. This is true, for example, of the facility for axial displacement between the inner container 9 and its bottom 12b, and between the inner container 9 and the outer container 10, which facility for axial displacement can be achieved other than by threaded engagement. Means other than the oblique plane 13 and the pointed cone 13a can be considered for the purpose of opening the ampoule 11.

The emptying sequence can also take place in the reverse order to that described above, i.e. first the bone cement component in powder form, and then the liquid bone cement component.

## Claims

1. Method for successively feeding batches of constituent components (A, B) into a mixing vessel (2) for the preparation of bone cement under vacuum, ***characterized*** in that, in an arbitrary sequence, a glass ampoule (11) containing a liquid bone cement component (A) surrounded by an inner container (9) communicating with the atmosphere and with the mixing vessel is opened so that the contents of the ampoule, under the effect of the partial vacuum inside the mixing vessel (2), can be sucked down into it, in that a space (15) formed by the spaces between the aforementioned inner container and by the outer container (10) that encloses it at least partially and filled with a bone cement component (B) in powder form is caused by displacement of the inner container (9) relative to the outer container (10) to move from a first position in which the space does not communicate with the atmosphere or the mixing vessel (2) to a second position in which it communicates with the atmosphere and the mixing vessel, so that the bone cement component (B) in powder form can be sucked down into the mixing vessel (2) under the effect of the partial vacuum inside it.

2. Arrangement for successively feeding batches of constituent components (A, B) into a mixing vessel (2) for the preparation of bone cement under vacuum, ***characterized*** in that the arrangement (1) comprises, on the one hand, an inner container (9) communicating with the atmosphere, which is so arranged as to enclose a glass ampoule (11) containing a liquid bone cement component (A) and as to communicate with the aforementioned mixing vessel (2), and comprising means (13; 13a) for opening the ampoule (11) so that its contents, under the effect of the partial vacuum inside the mixing vessel (2), can be sucked down into it and, on the other hand, an outer container (10) enclosing the inner container (9) at least partially and so arranged as to communicate with the mixing vessel (2), which outer container, together with the inner container (9), defines a space (15) filled with a certain quantity of a bone cement component (B) in powder form, and in which the inner container (9) is capable of displacement from a first position, in which sections of the inner container prevent communication between both the mixing vessel (2) and the atmosphere, to a second position, in which communication between the mixing vessel (2) on the one hand and the atmosphere on the other hand is open, so that the bone cement component (B) in powder form, under the effect of the partial vacuum inside the mixing vessel (2), can be sucked down into it, in conjunction with which feeding of the components (A, B) into the mixing vessel can take place in an arbitrary sequence.

3. Arrangement in accordance with Patent Claim 2, ***characterized*** in that the inner container (9) has a cylindrical part at the top, with a bottom (12b) capable of axial displacement relative to it, which bottom exhibits an opening (12d) between the interior of the container (9) and the atmosphere, and has a funnel-shaped part (9a) at the bottom adjacent to the cylindrical part, the downward-facing narrowest part of which is designed to communicate with an opening in the mixing vessel (2), in conjunction with which the glass ampoule (11), which has a downward-facing tip (11a) executed with a fractural impression, rests with the latter against an oblique plane (13) arranged inside the inner container (9), and is clamped between the former and the bottom (12b), so that as the latter is displaced successively downwards, the tip (11a) of the ampoule is broken off, and the liquid bone cement component (A) can be sucked down into the mixing vessel (2) by the partial vacuum inside it, and in that the outer container (10) has a cylindrical part and an upward-facing bottom (10b), in which the inner container (9) with its cylindrical part is mounted in such a way as to be capable of displacement between the aforementioned positions, and a funnel-shaped part (10a) adjacent to the cylindrical part, the downward-facing, narrowest part of which is intended to be connected to the aforementioned opening in the mixing vessel (2), in conjunction with which the funnel-shaped part (9a) of the inner container (9) is so arranged, in the first position, through contact with the funnel-shaped part (10a) of the outer container (10), as to prevent communication between the inner space (15) of the outer container (10) and the mixing vessel (2).

4. Arrangement in accordance with Patent Claim 2, ***characterized*** in that the inner container (9) has a cylindrical part at the top, with a bottom (12b) capable of axial displacement relative to it, which bottom exhibits an opening (12d) between the interior of the container (9) and the atmosphere, and has a funnel-shaped part (9a) at the bottom adjacent to the cylindrical part, the downward-facing narrowest part of which is designed to communicate with an opening in the mixing vessel (2), in conjunction with which the bottom (11a) of the glass ampoule rests against a cone (13a) arranged inside the inner container (9), and is clamped between the former and the bottom (12b), so that, as the latter is successively displaced downwards, the bottom (11b) of the ampoule is penetrated and broken, and the liquid bone cement component (A) can be sucked down into the mixing vessel (2) by the partial vacuum inside it, and in that the outer container (10) has a cylindrical part and an upward-facing bottom (10b), in which the inner container (9) with its cylindrical part is mounted in such a way as to be capable of displacement between the aforementioned positions, and a funnel-shaped part (10a) adjacent to the cylindrical part, the downward-facing, narrowest part of which is intended to be connected to the aforementioned opening in the mixing vessel (2), in conjunction with which the funnel-shaped part (9a) of the inner container (9) is so arranged, in the first position, through contact with the funnel-shaped part (10a) of the outer container (10), as to prevent communication between the inner space (15) of the outer container (10) and the mixing vessel (2).

5. Arrangement in accordance with Patent Claims 2, 3 or 4, ***characterized*** in that there is present between the bottom (10b) of the outer container (10) and the cylindrical part of the inner container (9) an opening (16) so arranged as to be closed in the aforementioned first position and as to be opened successively as the inner container (9) is displaced towards the second position.

6. Arrangement in accordance with anyone of the preceding Patent Claims 2-5, ***characterized*** in that a filter (14) is arranged beneath the ampoule (11), i.e. between it and the opening from the inner container (9) to the mixing vessel (2).

7. Arrangement in accordance with anyone of the preceding Patent Claims 2-6, ***characterized*** in that the inner container is executed in the vicinity of its funnel-shaped part (9a) with brush-like devices (9b) in contact with the funnel-shaped part (10a) of the outer container (10) and so arranged as to release the bone cement component (B) in powder form from the funnel-shaped part (10a).

## Patentansprüche

1. Verfahren zum aufeinanderfolgenden Zuführen von Chargen von Bestandteilen (A, B) in einen Mischbehälter (2) für die Herstellung von Knochenzement unter Vakuum,
**dadurch gekennzeichnet**, daß, in einer willkürlichen Reihenfolge eine einen flüssigen Knochenzementbestandteil (A) enthaltende und von einem mit der Atmosphäre und dem Mischbehälter in Verbindung stehenden inneren Behälter (9) umgebene Glasampulle (11) geöffnet wird, so daß die Inhalte der Ampulle unter der Wirkung des Partialvakuums im Innern des Mischbehälters (2) in diesen hinuntergesaugt werden können, daß ein Raum (15), gebildet durch die Räume zwischen dem oben genannten inneren Behälter und durch den äußeren Behälter (10), der diesen mindestens teilweise umschließt, und mit einem pulverförmigen Knochenzementbestand-teil (B) gefüllt, durch Verschiebung des inneren Behälters (9) relativ zu dem äußeren Behälter (10) von einer ersten Position, in der der Raum nicht mit der Atmosphäre oder dem Mischbehälter (2) in Verbindung steht, in eine zweite Position, in der er mit der Atmosphäre und dem Mischbehälter in Verbindung steht, bewegt wird, so daß der pulverförmige Knochenzementbestandteil (B) unter der Wirkung des Partialvakuums im Innern des Mischbehälters (2) in diesen hinuntergesaugt werden kann.

2. Anordnung zum aufeinanderfolgenden Zuführen von Chargen von Bestandteilen (A, B) in einen Mischbehälter (2) für die Herstellung von Knochenzement unter Vakuum,
**dadurch gekennzeichnet**, daß die Anordnung (1) einerseits einen inneren Behälter (9) umfaßt, der mit der Atmosphäre in Verbindung steht und so angeordnet ist, daß er eine einen flüssigen Knochenzementbestandteil (A) enthaltende Glasampulle (11) umschließt und mit dem oben genannten Mischbehälter (2) in Verbindung steht, und Mittel (13; 13a) für das Öffnen der Ampulle (11) umfaßt, so daß ihre Inhalte unter der Wirkung des Partialvakuums im Innern des Mischbehälters (2) hinunter in diesen gesaugt werden können, und andererseits einen äußeren Behälter (10) umfaßt, der den inneren Behälter (9) mindestens teilweise umschließt und so angeordnet ist, daß er mit dem Mischbehälter (2) in Verbindung steht, wobei der äußere Behälter, zusammen mit dem inneren Behälter (9), einen Raum (15) definiert, der mit einer bestimmten Menge eines pulverförmigen Knochenzementbestandteils (B) gefüllt ist, und in der der innere Behälter (9) von einer ersten Position, in der Bereiche des inneren Behälters eine Verbindung zwischen sowohl dem Mischbehälter (2) als auch der Atmosphäre verhindern, in eine zweite Position geschoben werden kann, in der die Verbindung zwischen dem Mischbehälter (2) auf der einen Seite und der Atmosphäre auf der anderen Seite offen ist, so daß der pulverförmige Knochenzementbestandteil (B) unter der Wirkung des Partialvakuums im Innern des Mischbehälters (2) in diesen hinuntergesaugt werden kann, wobei das Zuführen der Bestandteile (A, B) in den Mischbehälter in einer willkürlichen Reihenfolge stattfinden kann.

3. Anordnung gemäß Patentanspruch 2,
**dadurch gekennzeichnet**, daß der innere Behälter (9) an seiner Oberseite einen zylinderförmigen Teil mit einem Boden (12b) aufweist, der relativ zu diesem axial verschoben werden kann, wobei der Boden eine Öffnung (12d) zwischen dem Innern des Behälters (9) und der Atmosphäre aufweist, und der innere Behälter (9) an seiner Unterseite angrenzend an den zylinderförmigen Teil einen trichterförmigen Teil (9a) aufweist, dessen nach unten zeigender, engster Teil derart ausgestaltet ist, daß er mit einer Öffnung in dem Mischbehälter (2) in Verbindung steht, wobei die Glasampulle (11), die eine nach unten zeigende, mit einer Sollbruchstelle ausgestattete Spitze (11a) aufweist, mit letzterer gegen eine schiefe Ebene (13), angeordnet im Innern des inneren Behälters (9), ruht und zwischen ersterer und dem Boden (12b) eingeklemmt ist, so daß, wenn letztere nach und nach abwärts verschoben wird, die Spitze (11a) der Ampulle abgebrochen wird und der flüssige Knochenzementbestandteil (A) hinunter in den Mischbehälter (2) durch das Partialvakuum im Inneren des Mischbehälters gesaugt werden kann, wobei der äußere Behälter (10) einen zylinderförmigen Teil und einen nach oben zeigenden Boden (10b) aufweist, wobei der innere Behälter (9) mit seinem zylinderförmigen Teil in solch einer Weise angebracht ist, daß er zwischen den oben genannten Positionen verschoben werden kann, wobei ein trichterförmiger Teil (10a) angrenzend an den zylinderförmigen Teil, dessen nach unten zeigender, engster Teil dazu bestimmt ist, mit der oben genannten Öffnung in dem Mischbehälter (2) verbunden zu werden, wobei der trichterförmige Teil (9a) des inneren Behälters (9) so angeordnet ist, daß in der ersten Position durch einen Kontakt mit dem trichterförmigen Teil (10a) des äußeren Behälters (10) eine Verbindung zwischen dem inneren Raum (15) und dem äußeren Behälter (10) und dem Mischbehälter (2) verhindert wird.

4. Anordnung gemäß Patentanspruch 2,
**dadurch gekennzeichnet**, daß der innere Behälter (9) an der Oberseite einen zylinderförmigen Teil mit einem Boden (12b) aufweist, der relativ zu diesem axial verschoben werden kann, wobei der Boden eine Öffnung (12d) zwischen dem Inneren des Behälters (9) und der Atmosphäre aufweist, und der innere Behälter (9) an seiner Unterseite angrenzend an den zylinderförmigen Teil einen trichterförmigen Teil (9a) aufweist, dessen nach unten zeigender, engster Teil derart ausgestaltet ist, daß er mit einer Öffnung in dem Mischbehälter (2) in Verbindung steht, wobei der Boden (11a) der Glasampulle gegen einen Kegel (13a), angeordnet im Inneren des inneren Behälters (9) ruht und zwischen ersterem und dem Boden (12b) eingeklemmt ist, so daß, wenn letztere nach und nach nach unter verschoben wird, der Boden (11b) der Ampulle durchbohrt und zerbrochen wird und der flüssige Knochenzementbestandteil (A) in den Mischbehälter (2) durch das Partialvakuum im Inneren des Mischbehälters in diesen hinuntergesaugt werden kann, wobei der äußere Behälter (10) einen zylinderförmigen Teil und einen nach oben zeigenden Boden (10b) aufweist, wobei der innere Behälter (9) mit seinem zylinderförmigen Teil derart angebracht ist, daß er zwischen den oben genannten Positionen verschoben werden kann, wobei ein trichterförmiger Teil (10a), angrenzend an den zylinderförmigen Teil, dessen nach unten zeigender, engster Teil dazu bestimmt ist, mit der oben genannten Öffnung in dem Mischbehälter (2) verbunden zu werden, wobei der trichterförmige Teil (9a) des inneren Behälters (9) derart angeordnet ist, daß in der ersten Position durch einen Kontakt mit dem trichterförmigen Teil (10a) des äußeren Behälters (10) eine Verbindung zwischen dem inneren Raum (15) des äußeren Behälters (10) und dem Mischbehälter (2) verhindert wird.

5. Anordnung gemäß Patentansprüchen 2, 3 oder 4,
**dadurch gekennzeichnet**, daß zwischen dem Boden (10b) des äußeren Behälters (10) und dem zylinderförmigen Teil des inneren Behälters (9) eine Öffnung (16) vorhanden ist, die so angeordnet ist, daß sie in der oben genannten ersten Position geschlossen ist und nach und nach geöffnet wird, wenn der innere Behälter (9) zu der zweiten Position hin verschoben wird.

6. Anordnung gemäß einem der vorangehenden Patentansprüche 2-5,
**dadurch gekennzeichnet**, daß ein Filter (14) unterhalb der Ampulle (11) angeordnet ist, d.h., zwischen dieser und der Öffnung von dem inneren Behälter (9) zu dem Mischbehälter (2).

7. Anordnung gemäß einem der vorangehenden Ansprüche 2-6,
**dadurch gekennzeichnet**, daß der innere Behälter in der Nachbarschaft seines trichterförmigen Teils (9a) mit bürstenartigen Vorrichtungen (9b) ausgestattet ist, die in Kontakt mit dem trichterförmigen Teil (10a) des äußeren Behälters (10) stehen und so angeordnet sind, daß der pulverförmige Knochenzementbestandteil (B) von dem trichterförmigen Teil (10a) freigesetzt wird.

## Revendications

1. Méthode pour déverser successivement une pluralité de composants constitutifs (A,B) dans un vase de mélange (2) pour la préparation sous vide de ciment ostéoplastique , caractérisé :
en ce que, dans une séquence arbitraire, une ampoule de verre (11) contenant un composant (A) liquide pour ciment ostéoplastique entourée d'un conteneur intérieur (9) communiquant avec l'atmosphère et avec le vase de mélange, est ouverte de telle sorte que le contenu de l'ampoule, sous l'effet du vide partiel à l'intérieur du vase de mélange (2) puisse être aspiré vers le bas à l'intérieur de celui-ci , et
en ce qu'un espace (15) formé par les espaces entre le conteneur intérieur susmentionné et un conteneur extérieur (10) qui l'enferme au moins partiellement, et rempli par un composant pour ciment ostéoplastique (B) en poudre, résulte du déplacement du conteneur intérieur (9) par rapport au conteneur extérieur (10) depuis une première position dans laquelle l'espace ne communique pas avec l'atmosphère ou avec le vase de mélange (2), jusqu'à une seconde position dans laquelle il communique avec l'atmosphère et avec le vase de mélange, de telle sorte que le composant en poudre (B) pour ciment ostéoplastique puisse être aspiré vers le bas dans le vase de mélange (2) sous l'effet du vide partiel à l'intérieur de celui-ci.

2. Dispositif pour déverser successivement une pluralité de composants constitutifs (A,B) dans un vase de mélange (2) pour la préparation sous vide d'un ciment ostéoplastique, caractérisé :
en ce que le dispositif (1) comprend, d'une part, un conteneur intérieur (9), communiquant avec l'atmosphère, lequel est agencé de façon à enfermer une ampoule de verre (11) contenant un composant liquide pour ciment ostéoplastique (A) et de façon à communiquer avec le vase de mélange susmentionné (2), et comprenant des moyens (13 .13a) pour l'ouverture de l'ampoule (11) de telle sorte que son contenu, sous l'effet du vide partiel à l'intérieur du vase de mélange (12), puisse être aspiré vers le bas dans celui-ci et, d'autre part, un conteneur extérieur (10) renfermant le conteneur intérieur (9) au moins partiellement, et disposé de façon à communiquer avec le vase de mélange (2), lequel conteneur extérieur, ensemble avec le conteneur intérieur (9) définissent un espace (15) rempli d'une certaine quantité de composant en poudre (B) pour ciment ostéoplastique, et dans lequel le conteneur intérieur (9) est capable de déplacement depuis une première position, dans laquelle les parties du conteneur intérieur empêchent la communication entre à la fois le vase de mélange (2) et l'atmosphère, jusqu'à une seconde position, dans laquelle la communication entre le vase de mélange (2) d'une part et l'atmosphère d'autre part, est ouverte, de telle sorte que le composant en poudre (B) pour ciment ostéoplastique , sous l'effet du vide partiel à l'intérieur du vase de mélange (2) puisse être aspiré vers le bas dans celui-ci, grâce à quoi le déversement des composants (A,B) dans le vase de mélange peut avoir lieu dans une séquence arbitraire.

3. Dispositif selon la revendication 2, caractérisé :
en ce que le conteneur intérieur (9) a, à son sommet, une partie cylindrique, avec un fond (12b) capable de déplacement axial relativement à celui-ci, lequel fond présente une ouverture (12d) entre l'intérieur du conteneur (9) et l'atmosphère, et a sa partie de fond conformée en entonnoir (9a) au voisinage de la partie cylindrique, dont la partie la plus étroite regardant vers le bas est destinée à communiquer avec une ouverture dans le vase de mélange (2), grâce à quoi l'ampoule de verre (11), qui a une extrémité regardant vers le bas (11a) réalisée avec une amorce de cassure, est appliqué sur cette dernière contre un plan oblique (13) disposé en dedans du conteneur intérieur (9), et est fixée entre le précédent et le fond (12b), de sorte que, au fur et à mesure que le dernier est déplacé successivement vers le bas, le bout (11a) de l'ampoule est rompu et le composant liquide (A) pour ciment ostéoplastique peut être aspiré vers le bas dans le vase de mélange (2) par le vide partiel à l'intérieur de celui-ci, et
en ce que le conteneur extérieur (10) a une partie cylindrique et un fond regardant vers le haut (10b), dans lequel le conteneur intérieur (9) avec sa partie cylindrique est monté de façon à être capable de déplacement entre les positions susmentionnés, et une partie en forme d'entonnoir (10a) voisine de la partie cylindrique, regardant vers le bas, dont la partie la plus étroite est prévue pour être reliée à l'ouverture susmentionnée dans le vase de mélange (2), grâce à quoi la partie en forme d'entonnoir (9a) du conteneur intérieur (9) est disposée de façon à, dans le premier emplacement, par contact avec la partie en forme d'entonnoir (10a) du conteneur extérieur (10), empêcher la communication entre l'espace intérieur (15) du conteneur extérieur (10) et le vase de mélange (2).

4. Dispositif selon la revendication 2, caractérisé :
en ce que le conteneur intérieur (9) a, à son sommet, une partie cylindrique avec un fond (12b) capable de déplacement axial par rapport à celui-ci, lequel fond présente une ouverture (12d) entre l'intérieur du récipient (9) et l'atmosphère et a une partie en forme d'entonnoir (9a) à son fond voisin de la partie cylindrique, dont la partie la plus étroite regardant vers le bas, est destinée à communiquer avec une ouverture du vase de mélange (2), grâce à quoi le fond (11a) de l'ampoule de verre s'appuie contre un cône (13a) disposé à l'intérieur du conteneur intérieur (9), et est fixé entre le précédent et le fond (12b), de sorte que, au fur et à mesure que le second est successivement déplacé vers le bas, le fond (11b) de l'ampoule est enfoncé et rompu, et le composant liquide (A) pour ciment ostéoplastique peut être aspiré vers le bas dans le vase de mélange (2) par le vide partiel à l'intérieur de celui-ci, et
en ce que le conteneur extérieur (10) a une partie cylindrique et un fond regardant vers le haut (10b), dans laquelle le conteneur intérieur (9) avec sa partie cylindrique, est monté de façon à être capable de se déplacer entre les positions susmentionnées, et une partie (10a) en forme de entonnoir voisine de la partie cylindrique, regardant vers le bas, dont la partie la plus étroite, est destinée à être reliée à l'ouverture sumentionnée dans le vase de mélange (2), grâce à quoi la partie en forme d'entonnoir (9a) du conteneur intérieur (9) est disposé de façon à, dans la première position, par contact avec la partie en forme d'entonnoir (10a) du conteneur extérieur (10), empêcher la communication entre l'espace intérieur (15) du conteneur extérieur (10) et le vase de mélange (2).

5. Dispositif selon l'une quelconque des revendications 2, 3 ou 4, caractérisé :
en ce qu'une ouverture (16) se trouve disposée entre le fond (10b) du conteneur extérieur (10) et la partie cylindrique du conteneur intérieur (9), de façon à être dans la première position susmentionnée, et être ouverte successivement au fur et à mesure que le conteneur intérieur (9) se déplace dans la direction de la seconde position.

6. Dispositif selon l'une quelconque des revendications 2 à 5, caractérisé :
en ce qu'un filtre (14) est disposé sous l'ampoule (11), c'est-à-dire entre celle-ci et l'ouverture du conteneur intérieur (9) vers le vase de mélange (2).

7. Dispositif selon l'une quelconque des revendications 2 à 6, caractérisé :
en ce que le conteneur intérieur est réalisé au voisinage de sa partie conformée en entonnoir (9a), avec des dispositifs en forme de brosse (9b) en contact avec la partie en forme d'entonnoir (10a) du conteneur extérieur (10) et est disposé de façon à libérer le composant en poudre (B) pour ciment ostéoplastique à partir de la partie en forme d'entonnoir (10a).
